# EUROPEAN PATENT SPECIFICATION

(11) **EP 3 781 586 B1**
(45) Date of publication and mention of the grant of the patent: **17.04.2024**
(21) Application number: 19717920.3
(22) Date of filing: 18.04.2019
(51) Int. Cl.: C07K 7/06, C07K 7/18

(54) **A METHOD FOR PRODUCTION OF HIGH PURITY ICATIBANT**
VERFAHREN ZUR HERSTELLUNG VON HOCHREINEM ICATIBANT
PROCÉDÉ DE PRODUCTION D'UN ICATIBANT DE GRANDE PURETÉ

(30) Priority: 20.04.2018 EP 18168558
(43) Date of publication of application: 24.02.2021
(73) Proprietor: Fresenius Kabi iPSUM S.r.l., 20060 Cassina de' Pecchi - Milano (IT)
(72) Inventor: CABRI, Walter, 45010 Villadose (RO) (IT); POZZEBON, Alice, 45010 Villadose (RO) (IT); VIOLA, Angelo, 45010 Villadose (RO) (IT); RICCI, Antonio, 45010 Villadose (RO) (IT); BORGOGNO, Andrea, 45010 Villadose (RO) (IT); GURYANOV, Ivan, 35131 Padova (IT)
(74) Representative: Fresenius Kabi Deutschland GmbH
(86) International application number: PCT/EP2019/060035
(87) International publication number: WO 2019/202057

(56) References cited:
- WO-A1-2018/007930
- CN-A- 103 992 383
- A L ELLISON ET AL.: "Convenient synthesis of collagen-related tripeptides for segment condensation", BIOPOLYMERS, vol. 104, no. 6, 2015, pages 674-681, XP002784735, John Wiley & Sons, Inc. ISSN: 0006-3525
- DATABASE WPI Week 201807 Thomson Scientific, London, GB; AN 2017-861582 XP002791529, & CN 107 417 770 A (JIANGSU HANSOH PHARM GROUP CO LTD) 1 December 2017 (2017-12-01)

## Description

### Field of the invention

The present invention relates to peptidomimetic synthesis. In particular, it relates to processes for the manufacture of antagonists of bradykinin B2 receptor. More in particular, the present invention relates to a process for the manufacture of icatibant by fragment condensation and to the preparation of such fragments.

### Background of the invention

Hereditary angioedema (HAE) is a rare and potentially life-threatening disease caused by deficiency or dysfunction of C1 inhibitor (C1-INH) resulting in the overproduction of bradykinin. Patients with HAE experience significant morbidity and have an impaired quality of life, because of recurrent edema of the skin, larynx, gastrointestinal track, resulting in abdominal pain, nausea, etc. Mortality may result from laryngeal attacks and asphyxiation and has been estimated in up to 30% of previously undiagnosed patients. Abdominal attacks add to morbidity of disease and often increase the cost of the treatment due to hospitalizations and unnecessary surgeries (Kalra et al. Expert Opin. Orphan Drugs 2014, 2, 743-750; Cole et al. Ann. Pharmacother. 2013, 47, 49-55).

Among other pharmaceuticals for HAE treatment, such as ecallantide or C1-INH itself, icatibant is the only approved drug for self-administration by subcutaneous route to treat acute attacks of HAE. Icatibant was initially developed by Hoechst with the abbreviation HOE-140 as an antagonist to bradykinin B2 receptor (B2R) with specific and selective action. It inhibits bradykinin mediated vasodilation and vasopermeability and blocks edema formation in HAE (Kalra et al. cited above).

Icatibant is a synthetic peptidomimetic drug, containing ten amino acids, i.e. a decapeptide. Among them, five are non-proteinogenic, or unnatural, amino acids, four of which are so-called imino acids (i.e. amino acids containing a secondary amine group).
Icatibant is chemically designated as
(N-(N-(D-arginyl-L-arginyl-L-prolyl-(4R)-4-hydroxy-L-prolyl-glycyl-3-(2-thienyl)-L-alanyl-L-seryl)-(3R)-1,2,3,4-tetrahydroisoquinoline-3-carbonyl)-(2S,3aS,7aS)-octahydroindole-2-carbonyl)-L-arginine
and depicted herein below:

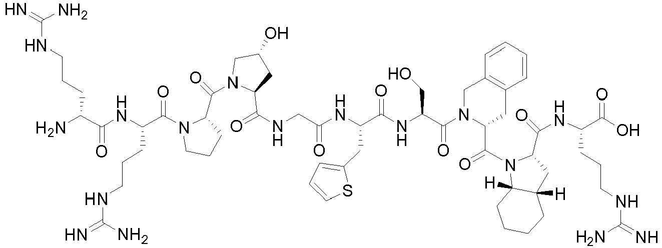

The structure of icatibant can be represented also as 
1 2 3 4 5 6 7 8 9 10
H-(D)Arg-Arg-Pro-Hyp-Gly-Thi-Ser-(D)Tic-Oic-Arg-OH
 wherein the numbering of residues starts with 1, indicating N-terminal (D)-arginine, and finishes with 10, indicating C-terminal arginine.

The unnatural amino acids of icatibant are (D)-arginine [(D)Arg], (L)-hydroxyproline (Hyp), β-2-thienylalanine (Thi), (R)-1,2,3,4-tetrahydroisoquinoline-3-carboxylic acid [(D)-Tic], and octahydroindole-2-carboxylic acid (Oic).

The pharmaceutical form of icatibant is the acetate salt thereof, which is the active ingredient in the commercialized product for injection named Firazyr^{®}.

The challenge in improving the icatibant synthesis processes lies in the nature of the peptide icatibant itself, which presents a number of imino acids (namely, proline (Pro), Hyp, (D)Tic and Oic) in its amino acid sequence, and in the consequent folding of icatibant into pseudocyclic conformation (β-turn structures).

In particular, when performing a number of steps of a solid phase synthesis, a number of side products with truncated sequences may form. These have to be removed and complicate the HPLC purification, thereby decreasing the yield of the final product.

The synthesis of full-length icatibant is described in US patent US5597803 (a-amino-acylated peptide) and US patent US5648333. Herein Fmoc or Boc solid phase peptide synthesis (SPPS) methods, starting from p-benzyloxybenzyl alcohol resin and using carbodiimide and racemization suppressing additives, such as HOBt are disclosed. US5597803 and US5648333 do not disclose the yield nor the purity of the final product.

Similarly, CN 102532267 (1), CN 104072585 (2) and IN2014CH05473 (3) disclose stepwise Fmoc SPPS on 2-chlorotrityl chloride resin (1) and Wang resin (2,3), by using various activating reagents. In these references the total yield of the peptide is declared to be 35%, 55% and 40%, respectively.

In another patent application, CN 103992383, a synthesis is disclosed which suggests the use of the dipeptide Boc-(D)Arg-Arg-OH·2HCl for the last coupling step of a stepwise Fmoc SPPS in order to decrease the amount of truncated sequences, such as des-D-Arg¹-icatibant and des-Arg²-icatibant. The total yield disclosed is 46%.

IN 201621021157 also discloses a stepwise Fmoc SPPS on a 2-chlorotrityl chloride resin (CTC resin). No information on yield is disclosed.

WO2018007930 describes a convergent fragment based synthesis of icatibant in liquid phase.

Thus, there remains a need to develop an efficient, simple and industrially viable process of preparing icatibant which could overcome the drawbacks of the prior art and provide icatibant in high yield, and with a favorable impurity profile, facilitating final purification.

The present inventors have developed an improved solid phase synthesis of icatibant which makes use of a convergent approach wherein two peptide fragments are coupled with the formation of an amide bond between amino acids 5 and 6, namely glycine (Gly) and β-2-thienylalanine (Thi). In particular, a strategy (5+5) is devised, or, alternatively, (4+5), or (3+5), or (2+5) strategies are used, wherein the C-terminal pentapeptide Thi-Ser-(D)Tic-Oic-Arg (fragment A) is coupled with a penta-, tetra-, tri- or di-peptide fragment with C-terminal glycine (fragment B), and wherein the possible addition of last N-terminal amino acids is accomplished by stepwise Fmoc SPPS to obtain full icatibant amino acids sequence.

### Object of the invention

It is an object of the present invention to provide an improved process in solid phase for the preparation of icatibant, or a pharmaceutically acceptable salt thereof, which results in crude icatibant in a high yield and a good impurity profile, facilitating final purification.

It is another object of the present invention to provide a process with higher yield and purity than achieved in the state of the art.

It is a further object of the present invention to provide useful peptide intermediates for the synthesis of icatibant, or a pharmaceutically acceptable salt thereof.

### Summary of the invention

The present invention provides a convergent method for the preparation of icatibant in solid phase synthesis, which comprises the step of coupling a first resin-bound -optionally protected-peptide fragment characterised by the amino acid sequence Thi-Ser-(D)Tic-Oic-Arg (fragment A), with a second -optionally protected- peptide fragment (fragment B) comprising a C-terminal glycine, optionally followed by one or more steps of elongating the resulting peptide with proper amino acids to complete the icatibant sequence.

Fragment A is bound to a resin, or solid support, at its C-terminal amino acid, i.e. arginine (Arg). The solid support is preferably selected from Wang resin, 2-chlorotrityl chloride resin or trityl chloride resin. Fragment A is preferably protected at its amino acids side- chains.

Fragment B has a C-terminal glycine and is optionally protected at its N-terminal amino group.

Fragment B is selected from the group consisting of
(D)Arg-Arg-Pro-Hyp-Gly-OH
(equivalent with N-terminal D-Arg of sequence Arg-Arg-Pro-Hyp-Gly-OH (SEQ ID No 1),
Arg-Pro-Hyp-Gly-OH (SEQ ID No 2),
Pro-Hyp-Gly-OH, and
Hyp-Gly-OH.
Preferably, fragment B is also protected at its amino acid side-chains.

Fragment B is characterized as providing a C-terminal glycine reacting with the N-terminal β-2-thienylalanine (Thi) residue of fragment A. Thereby an amide bond is formed, so that either icatibant full sequence, or a shorter peptide sequence, depending on the length of fragment B, is obtained. In the latter case, subsequent deprotection/coupling steps (i.e. steps of elongation) are performed in order to add the missing amino acids to complete the icatibant sequence in a stepwise SPPS fashion.

The obtained icatibant decapeptide, preferably side-chain protected, is deprotected and/or cleaved from the solid support to obtain crude icatibant. Such crude icatibant is optionally purified to obtain pure icatibant. The method of present invention provides icatibant in a high yield and a high purity.

Moreover, the present invention provides the intermediate fragments A and B and methods for their preparation.

### Detailed description of the invention

The present invention discloses a novel method for preparing icatibant with high yield and purity. In particular, it consists of a convergent approach on the solid phase. The preparation of short fragments and their subsequent coupling results in reduction of the amount of impurities due to increasing yields of the single coupling steps during the fragment synthesis. It also reduces the amount of the N-protected amino acids required for the single couplings.

The present invention provides a method for the preparation of icatibant, consisting of a convergent approach wherein optionally protected peptide fragments are coupled so that the C-terminal glycine carboxylic acid of a fragment B reacts with the N-terminal amino group of a fragment A, which is bound to a solid support. Such method substantially reduces the formation of impurities so that the obtained crude peptide can be easily purified to give icatibant in a high total yield.

In a further aspect, the present invention provides the required icatibant peptide fragments A and B and the method of preparing these. Such synthesis is performed either by SPPS or by Liquid Phase Peptide Synthesis (LPPS), both by using a coupling reagent system. Preferably it is performed by SPPS.

The term "peptide fragment" or "fragment", describes a peptide with a partial icatibant amino acid sequence, that can consist of two to nine amino acids. It can be optionally attached to a resin at its C-terminal amino acid. A peptide fragment can be protected or not protected.

The term "protected peptide fragment" or "protected fragment" describes a peptide fragment which can independently bear protecting groups at its amino acids side-chains, or side groups, and/or at its α-amino group.

A fragment, or peptide fragment, can be also indicated with a specific amino acids sequence, like
aa₁-aa₂-...-aaₙ
wherein aaₓ is the three letter code of the amino acid in position x.
When the fragment is indicated with
aa₁-aa₂-...-aaₙ-OH
it is intended that the C-terminal amino acid aaₙ has a free carboxylic acid.

The term "resin" or "solid support", describes a functionalized insoluble polymer to which an amino acid or a peptide fragment can be attached and which is suitable for amino acids elongation until the full sequence is obtained.

The SPPS can be defined as a process in which a peptide anchored by its C-terminal amino acid to a resin is assembled by the successive addition of the optionally protected amino acids constituting its sequence. It comprises loading a first α-amino-protected amino acid, or peptide, onto a resin and is followed by the repetition of a sequence of steps referred to as a cycle, or as a step of elongation, consisting of the cleavage of the α-amino protecting group and the coupling of the subsequent protected amino acid.

The formation of a peptide bond between two amino acids, or between an amino acid and a peptide, generally involves two steps. First the activation of the carboxyl group, then the nucleophilic attack of the amino group at the activated carboxylic group.

The cycle may be repeated sequentially until the desired sequence of the peptide is accomplished.

Finally, the peptide is deprotected and/or cleaved from the resin.

As a reference for SPPS, please see for instance Knud J. Jensen et al. (eds.), Peptide Synthesis and Applications, Methods in Molecular Biology, vol. 1047, Springer Science, 2013.

In a preferred aspect of present invention, an acid sensitive resin is used. More preferably, it is selected from Wang resin, 2-chlorotrityl chloride (CTC) resin and trityl chloride resin. Another preferred resin is 4-methylbenzhydryl bromide resin (MBH-Br).

Even more preferably, a Wang or a CTC resin is used for the preparation of fragment A and therefore also for coupling of fragment A with fragment B.

Preferably, a CTC resin is used for the preparation of fragment B.

In a preferred aspect of present invention, the loading of the first C-terminal amino acid onto the Wang resin is carried out after swelling of the resin in a suitable solvent, preferably DMF, filtering of the resin and adding to the resin the solution of protected amino acid with activating agent, such as a carbodiimide, in presence of dimethylaminopyridine (DMAP). In case of using a trityl chloride resin, and in particular the CTC resin, after swelling of the resin in a suitable solvent, preferably DCM, and filtering of the resin, a solution of protected amino acid with an organic base, preferably diisopropylethylamine (DIEA), is added.

In a preferred aspect of the present invention, after the first C-terminal amino acid has been loaded onto the resin, an additional step to block unreacted sites is performed to avoid truncated sequences and to prevent any side reactions. Such step is often referred to as "capping". Capping is achieved by a short treatment of the loaded resin with a large excess of a highly reactive unhindered reagent, which is chosen according to the unreacted sites to be capped. When using a Wang resin, the unreacted sites are hydroxyl groups, which are preferably capped by treatment with an acid derivative, such as an anhydride, in a basic medium, for instance with a DMF/DIEA/Ac₂O mixture, preferably with a 17/2/1 v/v/v composition.

When using a CTC resin, the unreacted sites are chlorines, which are preferably capped by treatment with an alcohol, optionally in a basic medium, for instance with a DCM/MeOH mixture, preferably with a 19/1 v/v composition, or a DCM/DIEA/MeOH mixture, preferably with a 17/2/1 v/v/v composition. Then, after washing with DCM, the resin is further treated with a DCM/Ac₂O mixture, optionally in the presence of DIEA, preferably with a DCM/DIEA/Ac₂O mixture, preferably with a 17/2/1 v/v/v composition, to cap the hydroxyl groups possibly resulting from the chlorine hydrolysis.

As an alternative to the loading of the first C-terminal amino acid, preloaded resins, which are Fmoc-protected L- or D-amino acids attached to a Wang/CTC resin, are used. Accordingly, Fmoc-Arg(Pbf)-Wang resin is preferably used for the synthesis of fragment A, and Fmoc-Gly-CTC resin is preferably used for the synthesis of fragment B. Another preferred preloaded resin for the synthesis of fragment A is Fmoc-Arg(Pbf)-CTC resin.

In a preferred aspect of present invention, the loading of the first C-terminal amino acid onto the resin is determined spectrophotometrically, as described for instance in Knud J. Jensen et al. (eds.), Peptide Synthesis and Applications, Methods in Molecular Biology, vol. 1047, Springer Science, 2013.

In a preferred aspect of present invention, each amino acid may be protected at its α-amino group and/or at its side-chain functional groups.

Preferably, the protecting group for amino acids α-amino groups used for the SPPS is of carbamate type. The most preferred protecting group is the 9-fluorenylmethoxycarbonyl (Fmoc) group, which can be removed under basic conditions.

The amino acids side-chain functional groups are optionally protected with groups which are generally stable during coupling steps and during α-amino protecting group removal, and which are themselves removable in suitable conditions. Such suitable conditions are generally orthogonal to the conditions in which the α-amino groups are de-protected.

In a preferred aspect of present invention, such side-chain protecting groups are specified per individual amino acid occurring in the icatibant sequence, as follows:
the arginine and (D)arginine guanidinium group is preferably protected by a protecting group selected from the group consisting of methoxy-2,3,6-trimethylbenzenesulfonyl (Mtr), 2,2,5,7,8-pentamethylchromanyl-6-sulfonyl (Pmc) and 2,2,4,6,7-pentamethyl-dihydrobenzofuran-5-sulfonyl (Pbf), all removable in acidic conditions; more preferably, the Pbf group is used;
the hydroxyproline (Hyp) hydroxyl group is preferably protected by a protecting group selected from the group consisting of trityl (Trt), tertbutyldimethylsilyl (TBDMS) and tertbutyl (tBu), all removable in acidic conditions; more preferably, the tBu group is used.

Analogously, the serine (Ser) hydroxyl group is preferably protected by a group selected from the group consisting of trityl (Trt), tertbutyldimethylsilyl (TBDMS) and tertbutyl (tBu); more preferably, the tBu group is used.

In a preferred aspect of the present invention, the coupling steps are performed in the presence of a coupling reagent.

Preferably, the coupling reagent is selected from the group consisting of N-hydroxysuccinimide (NHS), N,N'-diisopropylcarbodiimide (DIC), N,N'-dicyclohexylcarbodiimide (DCC), (Benzotriazol-1-yloxy)tripyrrolidinophosphonium hexafluorophosphate (PyBOP), 2-(7-Aza-1H-benzotriazole-1-yl)-1,1,3,3-tetramethyluronium hexafluorophosphate (HATU), 2-(1H-benzotriazole-1-yl)-1,1,3,3-tetramethyluronium hexafluorophosphate (HBTU) and ethyl-dimethylaminopropyl carbodiimide (EDC). More preferably, the reaction is carried out in the presence of N,N'-diisopropylcarbodiimide.

In a preferred aspect of present invention, the coupling steps are performed also in the presence of an additive. The presence of an additive, when used in the coupling reaction, reduces loss of configuration at the carboxylic acid residue, increases coupling rates and reduces the risk of racemization.

Preferably, the additive is selected from the group consisting of 1-hydroxybenzotriazole (HOBt), 2-hydroxypyridine N-oxide, N-hydroxysuccinimide (NHS), 1-hydroxy-7-azabenzotriazole (HOAt), endo-N-hydroxy-5-norbornene-2,3-dicarboxamide and ethyl 2-cyano-2-hydroxyimino-acetate (OxymaPure). More preferably, the reaction is carried out in the presence of 2-cyano-2-hyd roxyimino-acetate.

In a preferred aspect of the present invention, the coupling steps are performed in a solvent selected from the group consisting of DMF, DCM, THF, NMP, DMA or mixtures thereof. More preferably, the coupling is carried out in DMF when a Wang resin is used, and it is carried out in DCM when a CTC resin is used.

In a preferred aspect of present invention, the α-amino protecting groups are cleaved in basic conditions. In particular, the Fmoc protecting group is cleaved by treatment with a suitable secondary amine selected from the group consisting of piperidine, pyrrolidine, piperazine and DBU, preferably piperidine. Another preferred base for cleaving Fmoc groups is tert-butylamine. More preferably, Fmoc deprotection is carried out by using a 20% solution of piperidine in DMF.

In a preferred aspect of the present invention, once the desired peptide has been obtained according to SPPS as described above, the final deprotection and/or cleavage from the solid support is performed and either fragment B or icatibant is obtained. Preferably, such step is performed by using a specific mixture individualised for the resin used, in acidic or slightly acidic conditions.

When a CTC resin is used to synthesize the side-chain protected fragments mentioned above, the cleavage step is preferably performed by treatment using a mixture of HFIP:DCM (30:70 v/v) ) or 1-2 v/v % TFA solution in DCM. In particular, when a CTC resin is used in the preparation of fragment B, cleavage under these conditions does not remove the α-amino protecting group nor the side-chain protecting groups, thus yielding a protected fragment B.

In a preferred embodiment the side-chain protected peptide is isolated by pouring the cleavage mixture in a solvent selected from the group consisting of ethers, like diisopropyl ether (DIPE) and methyl-*tert*-butyl ether (MTBE), hydrocarbons, like petroleum ethers, pentane and hexane, esters, like ethyl acetate and isopropyl acetate, nitriles, like acetonitrile (ACN), or mixtures thereof. Preferably, the solvent is DIPE or MTBE. In another preferred embodiment the peptide is isolated by concentrating, or drying, *in vacuo* the cleavage mixture and then treating the residue with a solvent of the above list, preferably with DIPE or MTBE.

It is preferred that when a Wang or CTC resin is used in the final coupling to obtain icatibant sequence, the treatment is done with a cleavage mixture, comprising TFA and at least one scavenger, thereby providing both side-chains deprotection and cleavage from the resin. In particular, when a Wang resin is used in the preparation of fragment A, the final cleavage yields crude icatibant. Scavengers are substances, like, for instance, triisopropylsilane, anisole, thioanisole, 1,2-ethanedithiol, dithiothreitol, 2,2'-(ethylenedioxy)diethanethiol, dodecanethiol and phenol, capable of minimize modification or destruction of the sensitive deprotected side chains, such as those of arginine residues in icatibant sequence, in the cleavage environment.

Such cleavage/deprotection step is preferably performed by using a mixture of TFA/thioanisole/anisole/dodecanethiol, for instance with v/v/v/v 90/5/2/3 composition.

In a preferred embodiment the crude peptide is isolated by pouring the cleavage mixture in a solvent selected from the group consisting of ethers, like DIPE and MTBE, hydrocarbons, like petroleum ethers, pentane and hexane, esters, like ethyl acetate and isopropyl acetate, nitriles, like acetonitrile (ACN), or mixtures thereof. Preferably, the solvent is DIPE or MTBE.

In a preferred aspect of present invention, the crude peptide obtained by cleavage from the resin, be it fragment B or icatibant, is purified to increase its purity.

To this aim, a solution of the peptide is loaded onto an HPLC column with a suitable stationary phase, preferably C18 or C8 modified silica, and an aqueous mobile phase comprising an organic solvent, preferably acetonitrile or methanol, is passed through the column. A gradient of the mobile phase is applied, if necessary. The peptide with desired purity is collected and optionally lyophilized.

Accordingly, the present invention provides a convergent method for the preparation of icatibant in solid phase synthesis, wherein the method comprises the coupling of an optionally protected fragment A with sequence Thi-Ser-(D)Tic-Oic-Arg, with an optionally protected fragment B, as defined above, followed by optional amino acids coupling to complete the icatibant sequence.

The present invention provides the intermediate fragments A and B and methods for their preparation.

In one aspect, fragment A is icatibant peptide fragment (6-10) Thi-Ser-(D)Tic-Oic-Arg bound to a resin at its C-terminal arginine. The resin is selected from the group consisting of Wang resin, 2-chlorotrityl chloride (CTC) resin and trityl chloride resin. Preferably, the resin is Wang resin. In a preferred aspect, fragment A is protected at its side-chain functional groups with suitable protecting groups. In a more preferred aspect, fragment A is protected at the Arg residue with Pbf and at the Ser residue with tBu.

In an even more preferred aspect fragment A is fragment **1:**
H-Thi-Ser(*t*Bu)-(D)Tic-Oic-Arg(Pbf)-Wang resin **(1).**

Fragment A is prepared either by stepwise SPPS or by LPPS. For instance, fragment A is prepared in solid phase by starting with the loading on the resin of the protected C-terminal amino acid, i.e. arginine, and subsequently elongating the peptide chain by coupling the sequence amino acids one-by-one, by alternating coupling and α-amino group deprotection steps.

Preferably, Fmoc-Arg(Pbf)-OH is used for the initial loading of the resin, and stepwise Fmoc SPPS is used to complete the preparation of fragment A.

In another preferred aspect, fragment A is fragment **14:**
H-Thi-Ser(*t*Bu)-(D)Tic-Oic-Arg(Pbf)-CTC resin **(14).**

A preparation of fragment **1** and of fragment **14** is described in the Examples of present disclosure.

In another aspect, fragment B is selected from the group consisting of optionally protected (D)Arg-Arg-Pro-Hyp-Gly-OH, Arg-Pro-Hyp-Gly-OH, Pro-Hyp-Gly-OH and Hyp-Gly-OH.

In a preferred aspect, fragment B is protected at its α-amino group, even more preferably with
a Fmoc group.
Fragment B is preferably selected from the group consisting of
Fmoc-(D)Arg(Pbf)-Arg(Pbf)-Pro-Hyp(tBu)-Gly-OH (fragment **2),**
Fmoc-Arg(Pbf)-Pro-Hyp(tBu)-Gly-OH (fragment **3),**
Fmoc-Pro-Hyp(tBu)-Gly-OH (fragment **4),** and
Fmoc-Hyp(tBu)-Gly-OH (fragment **5).**

Most preferably, fragment B is Fmoc-(D)Arg(Pbf)-Arg(Pbf)-Pro-Hyp(tBu)-Gly-OH (fragment **2).**

A preparation of fragment **2** is described in the Examples of present disclosure.

In a preferred aspect, the convergent method for the preparation of icatibant comprises the coupling of optionally protected Thi-Ser-(D)Tic-Oic-Arg (fragment A) bound to a resin with optionally protected (D)Arg-Arg-Pro-Hyp-Gly-OH (fragment B), followed by deprotection/cleavage to obtain crude icatibant, which is optionally further purified.

Preferably, fragment A is H-Thi-Ser(*t*Bu)-(D)Tic-Oic-Arg(Pbf)-Wang resin (fragment **1),** fragment B is Fmoc-(D)Arg(Pbf)-Arg(Pbf)-Pro-Hyp(tBu)-Gly-OH (fragment **2),** and the coupling is performed in the presence of DIC/OxymaPure.

In such preferred aspect, the following α-amino protected icatibant sequence, attached to a Wang resin, is formed:
Fmoc-(D)Arg(Pbf)-Arg(Pbf)-Pro-Hyp(tBu)-Gly-Thi-Ser(*t*Bu)-(D)Tic-Oic-Arg(Pbf)-Wang resin **(6).**

The Fmoc protected resin bound peptide **(6)** is then treated to cleave the Fmoc group to obtain:
H-(D)Arg(Pbf)-Arg(Pbf)-Pro-Hyp(*t*Bu)-Gly-Thi-Ser(*t*Bu)-(D)Tic-Oic-Arg(Pbf)-Wang resin **(7).**

Finally, the resin bound peptide **(7)** is treated for simultaneous cleavage from the resin and cleavage of side-chain protecting groups to obtain crude icatibant.

In another preferred aspect, fragment A is H-Thi-Ser(*t*Bu)-(D)Tic-Oic-Arg(Pbf)-CTC resin (fragment **14),** fragment B is Fmoc-(D)Arg(Pbf)-Arg(Pbf)-Pro-Hyp(tBu)-Gly-OH (fragment **2),** and the coupling is performed in the presence of DIC/OxymaPure.

In such preferred aspect, the following α-amino protected icatibant sequence, attached to a CTC resin, is formed:

Fmoc-(D)Arg(Pbf)-Arg(Pbf)-Pro-Hyp(tBu)-Gly-Thi-Ser(*t*Bu)-(D)Tic-Oic-Arg(Pbf)-CTC resin **(15).**

The Fmoc protected resin bound peptide **(15)** is then treated to cleave the Fmoc group to obtain:
H-(D)Arg(Pbf)-Arg(Pbf)-Pro-Hyp(*t*Bu)-Gly-Thi-Ser(*t*Bu)-(D)Tic-Oic-Arg(Pbf)-CTC resin **(16).**

Finally, the resin bound peptide **(16)** is treated for simultaneous cleavage from the resin and cleavage of side-chain protecting groups to obtain crude icatibant.

In another preferred aspect, the convergent method for the preparation of icatibant of the present invention comprises the coupling of Thi-Ser-(D)Tic-Oic-Arg (fragment A) bound to a resin with optionally protected Arg-Pro-Hyp-Gly-OH (fragment B), followed by attachment of (D)Arg to obtain crude icatibant, which is optionally further purified.

Preferably, Fragment A is H-Thi-Ser(*t*Bu)-(D)Tic-Oic-Arg(Pbf)-Wang resin (fragment **1),** fragment B is Fmoc-Arg(Pbf)-Pro-Hyp(tBu)-Gly-OH (fragment **3),** and the coupling is performed in the presence of DIC/OxymaPure.

In such preferred aspect, the following sequence is formed:
Fmoc-Arg(Pbf)-Pro-Hyp(tBu)-Gly-Thi-Ser(*t*Bu)-(D)Tic-Oic-Arg(Pbf)-Wang resin (fragment **8).**

Fragment **8** is then treated to cleave the Fmoc group to obtain:
H-Arg(Pbf)-Pro-Hyp(*t*Bu)-Gly-Thi-ser(*t*Bu)-(D)Tic-Oic-Arg(Pbf)-Wang resin (fragment **9)**

(D)Arg is finally attached to fragment **9** to obtain α-amino protected icatibant sequence **6** (attached to a Wang resin), and the process is completed as described above to obtain crude icatibant.

In an additional preferred aspect, the convergent method for the preparation of icatibant of the present invention comprises the coupling of Thi-Ser-(D)Tic-Oic-Arg (fragment A) bound to a resin with optionally protected Pro-Hyp-Gly-OH (fragment B), followed by sequential attachment of Arg, and (D)Arg to obtain crude icatibant, which is optionally further purified.

Preferably, fragment A is H-Thi-Ser(*t*Bu)-(D)Tic-Oic-Arg(Pbf)-Wang resin (fragment **1),** fragment B is Fmoc-Pro-Hyp(tBu)-Gly-OH (fragment **4),** and the coupling is performed in the presence of DIC/OxymaPure.

In such preferred aspect, the following sequence is formed:
Fmoc-Pro-Hyp(tBu)-Gly-Thi-Ser(*t*Bu)-(D)Tic-Oic-Arg(Pbf)-Wang resin (fragment **10).**

Fragment **10** is then treated to cleave Fmoc group to obtain:
H-Pro-Hyp(*t*Bu)-Gly-Thi-Ser(*t*Bu)-(D)Tic-Oic-Arg(Pbf)-Wang resin (fragment **11)**

The elongation continues with Arg to obtain first fragment **8,** then fragment **9** after Fmoc cleavage, then the process is completed as described above to obtain crude icatibant.

In an additional preferred aspect, the convergent method for the preparation of Icatibant of the present invention comprises the coupling of Thi-Ser-(D)Tic-Oic-Arg (fragment A) bound to a resin with optionally protected Hyp-Gly-OH (fragment B), followed by sequential attachment of Pro, Arg, and (D)Arg to obtain crude Icatibant, which is optionally further purified.

Preferably, fragment A is H-Thi-Ser(*t*Bu)-(D)Tic-Oic-Arg(Pbf)-Wang resin (fragment **1),** fragment B is Fmoc-Hyp(tBu)-Gly-OH (fragment **5),** and the coupling is performed in the presence of DIC/OxymaPure.

In such preferred aspect, the following sequence is formed:
Fmoc-Hyp(tBu)-Gly-Thi-Ser(tBu)-(D)Tic-Oic-Arg(Pbf)-Wang resin (fragment **12).**

Fragment **12** is then treated to cleave Fmoc group to obtain:
H-Hyp(*t*Bu)-Gly-Thi-Ser(*t*Bu)-(D)Tic-Oic-Arg(Pbf)-Wang resin (fragment **13)**

The elongation continues analogously as described above, then the process is completed also as described above to obtain crude icatibant.

In another preferred aspect, the convergent method for the preparation of icatibant of the present invention comprises analogous couplings as in the hereabove described methods when fragment **14** is used instead of fragment **1.**

### Abbreviations

- SPPS: Solid Phase Peptide Synthesis
- LPPS: Liquid Phase Peptide Synthesis
- CTC: 2-chloro-trityl chloride
- Fmoc: 9-Fluorenylmethoxycarbonyl
- Boc: *Tert*-butyloxycarbonyl
- Trt: Trityl (triphenylmethyl)
- tBu: *Tert*-butyl
- Pbf: 2,2,4,6,7-Pentamethyl-dihydrobenzofuran-5-sulfonyl
- eq.: Equivalent
- h: hour/s
- min: minute/s
- HPLC: High Performance Liquid Chromatography
- DIEA: N,N-Diisopropylethylamine
- DMAP: 4-Dimethylaminopyridine
- TFA: Trifluoroacetic acid
- AczO: Acetic anhydride
- DMF: N,N-Dimethylformamide
- DMA: N,N-Dimethylacetamide
- DCM: Dichloromethane
- THF: Tetrahydrofuran
- NMP: N-Methyl-2-pyrrolidinone
- MTBE: Methyl-*tert*-butylether
- DIPE: Diisopropylether
- MeOH: Methanol
- HFIP: Hexafluoro-2-propanol
- TBDMS: Tert-butyl-dimethyl-silyl
- DIC: N,N'-Diisopropylcarbodiimide
- DCC: N,N'-Dicyclohexylcarbodiimide
- EDC: N-(3-dimethylaminopropyl)-N'-ethylcarbodiimide
- HOBt: 1-Hydroxybenzotriazole
- HOAt: 1-Hydroxy-7-azabenzotriazole
- NHS: N-Hydroxysuccinimide
- TBTU: O-(Benzotriazol-1-yl)-N,N,N',N'-tetramethyluronium tetrafluoroborate
- HBTU: O-(Benzotriazol-1-yl)-N,N,N',N'-tetramethyluronium hexafluorophosphate
- HATU: 2-(7-Aza-1H-benzotriazole-1-yl)-1,1,3,3-tetramethyluronium hexafluorophosphate
- PyBOP: (Benzotriazol-1-yloxy)tripyrrolidinophosphonium hexafluorophosphate
- OxymaPure: Ethyl 2-cyano-2-hydroxyimino-acetate

### Examples

Detailed experimental parameters suitable for the preparation of icatibant according to the present invention are provided by the following examples, which are intended to be illustrative and not limiting of all possible embodiments of the invention.

Unless otherwise noted, all materials, solvents and reagents were obtained from commercial suppliers, of the best grade, and used without further purification.

### Example 1

### Preparation of icatibant via (5+5) strategy

### Step 1. Preparation of fragment A on Wang resin:

### H-Thi-Ser(tBu)-(D)Tic-Oic-Arg(Pbf)-Wang resin (1)

Synthesis of peptide fragment **1** was carried out at room temperature by stepwise SPPS using Wang resin (250 mg, 0.75 mmol/g). After swelling of the resin in 2 ml of DMF, Fmoc-Arg(Pbf)-OH, DIC and DMAP (30, 15 and 0.6 eq. respectively, referred to the loading of the resin) in DMF were added. The unreacted hydroxyl groups of the resin were capped by treatment with DMF/DIEA/Ac₂O mixture (v/v/v 17/2/1) for 30 minutes. Fmoc deprotection was performed with 20% solution of piperidine in DMF (2 × 2 ml, 5 min and 15 min). Then, the resin was washed with DMF (4 × 2 ml). The loading of the resin was checked spectrophotometrically by UV adsorption measurement and was found to be 0.3 mmol/g. Fmoc-Thi-OH, Fmoc-Ser(tBu)-OH, Fmoc-(D)Tic-OH, Fmoc-Oic-OH (3 eq. each, referred to the loading of the resin) were each pre-activated with DIC (30 mg, 0.23 mmol) and ethyl 2-cyano-2-hydroxyimino-acetate (OxymaPure, 32 mg, 0.23 mmol) for 3 minutes and consecutively coupled to the resin for 60 min each time to finally get Fmoc-protected 5-mer peptide. The intermediate and the final Fmoc deprotections were carried out with 20% solution of piperidine in DMF (2 × 2 ml, 5 min and 15 min). After the completion of the synthesis the resin was washed with DMF (4 × 2 ml) to obtain the title compound.

### Step 1bis. Preparation of fragment A on CTC resin:

### H-Thi-Ser(tBu)-(D)Tic-Oic-Arg(Pbf)-CTC-Resin (14)

Synthesis of peptide fragment **14** was carried out at room temperature by stepwise SPPS using CTC resin (5 g, 1.6 mmol/g). After swelling of the resin in 40 ml of DCM, Fmoc-Arg(Pbf)-OH and DIEA (3 and 3 eq. respectively, referred to the loading of the resin) in DCM (8 vol) were added. The unreacted chloride groups of the resin were capped by treatment with DCM/MeOH (29 and 1 ml respectively) mixture for 15 min. The unreacted hydroxyl groups of the resin were capped by treatment with DCM/Ac₂O (28 and 2.5 ml respectively) mixture for 15 min. Fmoc deprotection was performed with 20% solution of piperidine in DMF (2 × 30 ml, 2 × 10 min). Then, the resin was washed with DMF (4 × 30 ml). The loading of the resin was checked spectrophotometrically by UV adsorption measurement and was found to be 1.1 mmol/g. Fmoc-Thi-OH, Fmoc-Ser(tBu)-OH, Fmoc-(D)Tic-OH, Fmoc-Oic-OH (3 eq. each, referred to the loading of the resin) were each pre-activated with DIC (2.55 ml, 3 eq) and ethyl 2-cyano-2-hydroxyimino-acetate (OxymaPure, 2.34 g, 3 eq) for 5 minutes and consecutively coupled to the resin for 90 min each time to finally get Fmoc-protected 5-mer peptide. The intermediate and the final Fmoc deprotections were carried out with 20% solution of piperidine in DMF (2 × 30 ml, 2 × 10 min). After the completion of the synthesis the resin was washed with DCM (3 × 30 ml) to obtain the title compound.

### Step 2. Preparation of fragment B

### Fmoc-(D)Arg(Pbf)-Arg(Pbf)-Pro-Hyp(tBu)-Gly-OH (2)

Synthesis of peptide fragment **2** was carried out at room temperature by stepwise SPPS using 2-chlorotrityl chloride resin (250 mg, 1.6 mmol/g). After swelling of the resin using 5 ml of DCM, Fmoc-Gly-OH and DIEA (0.75 and 1.25 eq. respectively, referred to the loading of the resin) in DCM were added. The reaction mixture was stirred for 1 hour and the unreacted sites of the resin were capped using a DCM/DIEA/MeOH mixture (v/v/v 17/2/1) for 30 minutes. Then, after washing with DCM (3 × 5 ml), the resin was treated with DCM/DIEA/Ac₂O mixture (v/v 17/2/1) for 30 minutes. Fmoc deprotection was performed with 20% solution of piperidine in DMF (2 × 2 ml, 5 min and 15 min). Then, the resin was washed with DMF (4 × 2 ml). The loading of the resin was checked by UV adsorption measurement and was found to be 0.67 mmol/g. Then, Fmoc-Hyp(tBu)-OH, Fmoc-Pro-OH, Fmoc-Arg(Pbf)-OH and Fmoc-(D)Arg(Pbf)-OH (3 eq. each, referred to the loading of the resin) were pre-activated by DIC (63 mg, 0.5 mmol) and OxymaPure (71 mg, 0.5 mmol) for 3 minutes and coupled to the resin for 60 min each time to finally get Fmoc-protected 5-mer peptide. Dry peptide resin was suspended in 4 ml of a mixture HFIP:DCM (v/v 30:70) and stirred for 1 h. The solution was filtered and the procedure was repeated 3 times. The organic solutions containing the cleaved protected peptide were collected and dried *in vacuo.* The crude peptide fragment **2** was then suspended in 10 ml of MTBE, filtered, washed twice with 5 ml of MTBE and dried.

According to this same methodology, fragments **3, 4** and **5,** as defined in the *Detailed description of the invention part* of present disclosure, are prepared, by interrupting the elongation of the peptide at the desired amino acid sequence.

### Step 3. Coupling of peptide fragments 1 and 2

### Preparation of H-(D)Arg(Pbf)-Arg(Pbf)-Pro-Hyp(tBu)-Gly-Thi-Ser(tBu)-(D)Tic-Oic-Arg(Pbf)-Wang resin (7)

A solution of peptide fragment **2** (80 mg, 0.058 mmol, 1.3 eq), as prepared in step 2, in 2 ml of DMF, containing 1.3 eq. of both OxymaPure and DIC, was added to 0.045 mmol (1 eq) of resin bound peptide fragment **1,** as obtained in step 1. The reaction mixture was stirred for 36 h at room temperature, then it was filtered and the resin was washed with DMF (3×2 ml) to finally obtain intermediate **6.** Fmoc protective group was removed by 20% solution of piperidine in DMF (2×2 ml, 5 min and 15 min) and the resin was washed again with DMF (2×2 ml), DCM (2×2 ml) and dried, to obtain the title compound.

### Step 3bis. Coupling of peptide fragments 14 and 2

### Preparation of H-(D)Arg(Pbf)-Arg(Pbf)-Pro-Hyp(tBu)-Gly-Thi-Ser(tBu)-(D)Tic-Oic-Arg(Pbf)-CTC resin (16)

A solution of peptide fragment **2,** (900 mg 0.65 mmol, 1.3 eq) as prepared in step 2, in 4.5 ml of DCM, containing 1.3 eq. of both OxymaPure and DIC, was added to 0.5 mmol (1 eq.) of resin bound peptide fragment **14,** as obtained in step 1bis. The reaction mixture was stirred for 18 h at room temperature, then it was filtered and the resin was washed with DCM (3×30 ml) to finally obtain intermediate **15.** Fmoc protective group was removed by 20% solution of piperidine in DMF (2×30 ml, 2 × 10 min) and the resin was washed again with DMF (4×30 ml), DCM (3×30 ml) and dried, to obtain the title compound.

### Step 4. Cleavage of icatibant from the resin

### Preparation of crude icatibant

Dry resin bound peptide fragment **7** (0.045 mmol), as obtained in step 3, was suspended in 2 ml of TFA/thioanisole/anisole/dodecanethiol (v/v/v/v 90/5/2/3) and stirred for 4 h. Then the resin was filtered and washed with 1 ml of TFA. The organic solutions were collected and dried *in vacuo.* The solid residue was washed with MTBE and dried to get crude icatibant with overall yield 80% and HPLC purity 83%.

### Step 4bis. Cleavage of icatibant from the resin

### Preparation of crude icatibant

Dry resin bound peptide fragment **16,** as obtained in step 3bis, was suspended in 4 ml of TFA/thioanisole/anisole/dodecanethiol (v/v/v/v 90/5/2/3) and stirred for 4 h. Then the resin was filtered and washed with 2 ml of TFA. The organic solutions were collected and poured in 20 ml of DIPE. The obtained suspension was stirred for 30 min at 5°C, filtered, the cake washed twice with DIPE (1 ml each wash) and dried to get crude icatibant with overall yield of 79% and HPLC purity of 85.4%.

### Step 5. Purification of icatibant.

The crude icatibant obtained in step 4, was dissolved in 1 ml of water and purified using 250×21.2 mm C18 column using 0.1% TFA/acetonitrile mobile phase. The fractions with purity greater than 99% were collected. Counter-ion exchange was carried out using 250×21.2 mm C18 column with mobile phase containing ammonium acetate. The fractions with purity greater than 99% were collected and lyophilized to give 43 mg of icatibant acetate.

HPLC purity: 99.7%, total yield 70%.

### Step 5bis. Purification of icatibant.

The crude icatibant obtained in step 4bis, was dissolved in 10 ml of water and purified using 250×21.2 mm C18 column using 0.1% TFA/acetonitrile mobile phase. The fractions with purity greater than 99% were collected. Counter-ion exchange was carried out using 250×21.2 mm C18 column with mobile phase containing ammonium acetate. The fractions with purity greater than 99% were collected and lyophilized to give 500 mg of icatibant acetate.

HPLC purity: 99.7%, total yield 67.2%.

## Claims

1. A method for the preparation of icatibant, wherein the method comprises the step of coupling a first resin-bound peptide fragment A **characterised by** the sequence Thi-Ser-(D)Tic-Oic-Arg, with a second peptide fragment B comprising a C-terminal glycine, optionally followed by one or more steps of elongation of the resulting peptide to complete the icatibant sequence.

2. The method according to claim 1, wherein resin-bound peptide fragment A is protected at the Arg residue with Pbf and at the Ser residue with tBu.

3. The method according to claim 1 or 2, wherein the resin is a Wang resin or a 2-chlorotrityl chloride resin.

4. The method according to any of the preceding claims, wherein fragment A is H-Thi-Ser(*t*Bu)-(D)Tic-Oic-Arg(Pbf)-Wang resin **(1)** or H-Thi-Ser(*t*Bu)-(D)Tic-Oic-Arg(Pbf)- 2-chloro-trityl chloride resin **(14).**

5. The method according to any of the preceding claims, wherein fragment B is selected from the group consisting of optionally protected (D)Arg-Arg-Pro-Hyp-Gly-OH, Arg-Pro-Hyp-Gly-OH, Pro-Hyp-Gly-OH and Hyp-Gly-OH.

6. The method according to claim 5, wherein fragment B is selected from the group consisting of
Fmoc-(D)Arg(Pbf)-Arg(Pbf)-Pro-Hyp(tBu)-Gly-OH **(2),**
Fmoc-Arg(Pbf)-Pro-Hyp(tBu)-Gly-OH **(3),**
Fmoc-Pro-Hyp(tBu)-Gly-OH **(4),** and
Fmoc-Hyp(tBu)-Gly-OH **(5).**

7. The method according to claim 5 or 6, wherein fragment B is prepared by Solid Phase Peptide Synthesis on a 2-chloro-trityl chloride resin.

8. The method according to any one of claims 1 to 6, wherein the coupling is carried out in the presence of N,N'-diisopropylcarbodiimide and ethyl 2-cyano-2-hydroxyimino-acetate.

9. The method according to claim 6, wherein
fragment A is
H-Thi-Ser(*t*Bu)-(D)Tic-Oic-Arg(Pbf)-Wang resin **(1),** or
H-Thi-Ser(*t*Bu)-(D)Tic-Oic-Arg(Pbf)-CTC-Resin **(14),**
fragment B is Fmoc-(D)Arg(Pbf)-Arg(Pbf)-Pro-Hyp(tBu)-Gly-OH **(2),** and
the coupling is carried out in the presence of N,N'-diisopropylcarbodiimide and ethyl 2-cyano-2-hydroxyimino-acetate.

10. The method according to any one of claims 1 to 9, wherein the method further comprises deprotection, preferably deprotection of the N-terminal Fmoc and the potentially protected side groups, and cleavage of icatibant from the resin.

11. The method according to any one of claims 1 to 10, wherein the method further comprises purification of icatibant by chromatography.

12. A fragment selected from the group consisting of
Fmoc-(D)Arg(Pbf)-Arg(Pbf)-Pro-Hyp(*t*Bu)-Gly-OH **(2),** and
Fmoc-Arg(Pbf)-Pro-Hyp(tBu)-Gly-OH **(3).**

13. Use of the fragment according to claim 12 in the preparation of icatibant.

## Patentansprüche

1. Verfahren zur Herstellung von Icatibant, wobei das Verfahren den Schritt des Koppelns eines ersten harzgebundenen Peptidfragments A, das durch die Sequenz Thi-Ser-(D)Tic-Oic-Arg gekennzeichnet ist, mit einem zweiten ein C-terminales Glycin umfassenden Peptidfragment B umfasst, gegebenenfalls gefolgt von einem oder mehreren Schritten der Verlängerung des resultierenden Peptids zur Vervollständigung der Icatibant-Sequenz.

2. Verfahren nach Anspruch 1, wobei das harzgebundene Peptidfragment A an dem Arg-Rest mit Pbf und an dem Ser-Rest mit tBu geschützt ist.

3. Verfahren nach Anspruch 1 oder 2, wobei das Harz ein Wang-Harz oder ein 2-Chlortritylchloridharz ist.

4. Verfahren nach einem der vorhergehenden Ansprüche, wobei Fragment A H-Thi-Ser(tBu)-(D)Tic-Oic-Arg(Pbf)-Wang-Harz **(1)** oder H-Thi-Ser(tBu)-(D)Tic-Oic-Arg(Pbf)-2-Chlortritylchloridharz **(14)** ist.

5. Verfahren nach einem der vorhergehenden Ansprüche, wobei Fragment B aus der Gruppe ausgewählt ist, die aus gegebenenfalls geschütztem (D)Arg-Arg-Pro-Hyp-Gly-OH, Arg-Pro-Hyp-Gly-OH, Pro-Hyp-Gly-OH und Hyp-Gly-OH besteht.

6. Verfahren nach Anspruch 5, wobei Fragment B aus der Gruppe ausgewählt ist, die aus Folgendem besteht:
**Fmoc-(D)Arg(Pbf)-Arg(Pbf)-Pro-Hyp(tBu)-Gly-OH (2),**
Fmoc-Arg(Pbf)-Pro-Hyp(tBu)-Gly-OH **(3),**
Fmoc-Pro-Hyp(tBu)-Gly-OH **(4)** und
Fmoc-Hyp(tBu)-Gly-OH **(5).**

7. Verfahren nach Anspruch 5 oder 6, wobei Fragment B durch Festphasen-Peptidsynthese auf einem 2-Chlortritylchloridharz hergestellt wird.

8. Verfahren nach einem der Ansprüche 1 bis 6, wobei die Kopplung in Anwesenheit von N,N'-Diisopropylcarbodiimid und Ethyl-2-cyano-2-hydroxyiminoacetat durchgeführt wird.

9. Verfahren nach Anspruch 6, wobei
Fragment A H-Thi-Ser(tBu)-(D)Tic-Oic-Arg(Pbf)-Wang-Harz **(1)** oder
H-Thi-Ser(*t*Bu)-(D)Tic-Oic-Arg(Pbf)-CTC-Harz **(14)** ist,
Fragment B Fmoc-(D)Arg(Pbf)-Arg(Pbf)-Pro-Hyp(tBu)-Gly-OH ist **(2)** and wobei das Koppeln in Anwesenheit von N,N'-Diisopropylcarbodiimid und Ethyl-2-cyano-2-hydroxyiminoacetat durchgeführt wird.

10. Verfahren nach einem der Ansprüche 1 bis 9, wobei das Verfahren ferner die Entschützung, vorzugsweise die Entschützung des N-terminalen Fmoc und der potentiell geschützten Seitengruppen, und die Abspaltung von icatibant aus dem Harz umfasst.

11. Verfahren nach einem der Ansprüche 1 bis 10, wobei das Verfahren ferner die Reinigung von Icatibant durch Chromatographie umfasst.

12. Fragment ausgewählt aus der Gruppe bestehend aus
Fmoc-(D)Arg(Pbf)-Arg(Pbf)-Pro-Hyp(*t*Bu)-Gly-OH **(2)** und
Fmoc-Arg(Pbf)-Pro-Hyp(tBu)-Gly-OH **(3).**

13. Verwendung des Fragments nach Anspruch 12 bei der Herstellung von I catibant.

## Revendications

1. Procédé pour la préparation d'icatibant, le procédé comprenant l'étape de couplage d'un premier fragment peptidique A lié à une résine **caractérisé par** la séquence Thi-Ser-(D)Tic-Oic-Arg, avec un second fragment peptidique B comprenant une glycine C-terminale, éventuellement suivie d'une ou plusieurs étapes d'élongation du peptide résultant pour compléter la séquence de l'icatibant.

2. Procédé selon la revendication 1, dans lequel le fragment peptidique A lié à une résine est protégé au niveau du résidu Arg avec Pbf et au niveau du résidu Ser avec tBu.

3. Procédé selon la revendication 1 ou 2, dans lequel la résine est une résine Wang ou une résine de chlorure de 2-chlorotrityle.

4. Procédé selon l'une quelconque des revendications précédentes, dans lequel le fragment A est H-Thi-Ser(*t*Bu)-(D)Tic-Oic-Arg(Pbf)-résine Wang **(1)** ou H-Thi-Ser(*t*Bu)-(D)Tic-Oic-Arg(Pbf)-résine de chlorure de 2-chlorotrityle **(14).**

5. Procédé selon l'une quelconque des revendications précédentes, dans lequel le fragment B est choisi dans le groupe constitué par (D)Arg-Arg-Pro-Hyp-Gly-OH, Arg-Pro-Hyp-Gly-OH, Pro-Hyp-Gly-OH et Hyp-Gly-OH éventuellement protégés.

6. Procédé selon la revendication 5, dans lequel le fragment B est choisi dans le groupe constitué par
Fmoc-(D)Arg(Pbf)-Arg(Pbf)-Pro-Hyp(tBu)-Gly-OH **(2),**
Fmoc-Arg(Pbf)-Pro-Hyp(*t*Bu)-Gly-OH **(3),**
Fmoc-Pro-Hyp(*t*Bu)-Gly-OH **(4)** et
Fmoc-Hyp(*t*Bu)-Gly-OH **(5).**

7. Procédé selon la revendication 5 ou 6, dans lequel le fragment B est préparé par synthèse peptidique en phase solide sur une résine de chlorure de 2-chlorotrityle.

8. Procédé selon l'une quelconque des revendications 1 à 6, dans lequel le couplage est effectué en présence de *N,N*'-diisopropylcarbodiimide et de 2-cyano-2-hydroxyiminoacétate d'éthyle.

9. Procédé selon la revendication 6, dans lequel
le fragment A est H-Thi-Ser(*t*Bu)-(D)Tic-Oic-Arg(Pbf)-résine Wang **(1)** ou
H-Thi-Ser(*t*Bu)-(D)Tic-Oic-Arg(Pbf)-résine de CTC **(14),**
le fragment B est Fmoc-(D)Arg(Pbf)-Arg(Pbf)-Pro-Hyp(*t*Bu)-Gly-OH **(2)** et
le couplage est effectué en présence de *N,N*'-diisopropylcarbodiimide et de 2-cyano-2-hydroxyiminoacétate d'éthyle.

10. Procédé selon l'une quelconque des revendications 1 à 9, le procédé comprenant en outre une déprotection, de préférence une déprotection du Fmoc N-terminal et des groupes latéraux potentiellement protégés, et le clivage d'icatibant de la résine.

11. Procédé selon l'une quelconque des revendications 1 à 10, le procédé comprenant en outre la purification d'icatibant par chromatographie.

12. Fragment choisi dans le groupe constitué par
Fmoc-(D)Arg(Pbf)-Arg(Pbf)-Pro-Hyp(*t*Bu)-Gly-OH **(2)** et
Fmoc-Arg(Pbf)-Pro-Hyp(*t*Bu)-Gly-OH **(3).**

13. Utilisation du fragment selon la revendication 12 dans la préparation d'icatibant.
